# FASCICULE DE BREVET EUROPEEN

(11) **EP 0 761 199 B1**
(45) Date de publication et mention de la délivrance du brevet: **21.01.1998**
(21) Numéro de dépôt: 96401644.8
(22) Date de dépôt: 23.07.1996
(51) Int. Cl.: A61K 7/06

(54) **Composition cosmétique à pouvoir fixant et/ou conditionneur contenant un copolymère acrylique particulier**
Kosmetische Zusammensetzung die zur Fixierung und/oder Festigung geeignet ist und die ein bestimmtes Acrylcopolymer enthält
Cosmetic composition having fixing and conditioning properties containing a particular acrylic copolymer

(30) Priorité: 11.08.1995 FR 9509774
(43) Date de publication de la demande: 12.03.1997
(73) Titulaire: L'OREAL, 75008 Paris (FR)
(72) Inventeur: Dupuis, Christine, 75018 Paris (FR)
(74) Mandataire: Andral, Christophe André Louis

(56) Documents cités:
- EP-A- 0 379 082
- EP-A- 0 590 604
- DE-A- 4 314 305
- FR-A- 2 351 135
- CHEMICAL ABSTRACTS, vol. 89, no. 14, 2 Octobre 1978 Columbus, Ohio, US; abstract no. 117546m, page 517; colonne r; XP002000482 & JP-A-53 012 429 (ROHM AND HAAS CO.)

## Description

L'invention concerne une composition cosmétique facilement pulvérisable et/ou vaporisable comprenant dans un milieu aqueux ou hydroalcoolique cosmétiquement acceptable, au moins un polymère fixant et/ou conditionneur et au moins un copolymère acrylique particulier, ainsi que l'utilisation de ce copolymère particulier pour améliorer la vaporisation ou la pulvérisation de la composition comprenant au moins un polymère fixant et/ou conditionneur.

Les compositions capillaires à pulvériser sur les cheveux sont essentiellement constituées d'une solution le plus souvent hydroalcoolique et d'un polymère en mélange éventuellement avec divers adjuvants cosmétiques. Cette solution est conditionnée soit dans un flacon pompe, soit dans un récipient aérosol approprié qui est mis sous pression à l'aide d'un gaz propulseur.

Pour des raisons essentiellement écologiques, on cherche à réduire les composés volatils à la pression atmosphérique, dits COV (Composés Organiques Volatils) qui sont présents dans les compositions. Les COV sont principalement les propulseurs et certains solvants tels que l'éthanol.

Ainsi, la législation de certains pays a imposé un taux maximum de COV dans les compositions cosmétiques en aérosol. Pour diminuer la quantité de COV, on a essayé de remplacer les solvants tels que l'éthanol par de l'eau.
Ces modifications peuvent entraîner des problèmes en particulier sur la qualité de la pulvérisation du spray. Les particules liquides pulvérisées ne sont pas fines, le spray est souvent pincé, c'est à dire non diffus, et la formation d'une légère mousse et/ou de bulles peut se produire.
La pulvérisation étant un élément essentiel pour la qualité finale d'une composition à pulvériser sur les cheveux, il est primordial de remédier à ces inconvénients pour obtenir une bonne répartition sur l'ensemble de la chevelure et une fixation et/ou un conditionnement correct des cheveux.

La demande EP-A- 590604 décrit une composition aqueuse à base d'un copolymère anionique acrylique et des compositions de sprays capillaires comprenant cette composition aqueuse à base de copolymère anionique acrylique. Cette composition à base de polymère est compatible avec de nombreux solvants. Elle présente de bonnes propriétés cosmétiques. Cependant, l'aspect des sprays obtenus n'est pas encore satisfaisant.

La demanderesse vient maintenant de découvrir, de façon inattendue et surprenante, qu'en associant les polymères habituellement utilisés dans les compositions de maintien et/ou de conditionnement de la coiffure avec le copolymère anionique particulier décrit ci-dessus, il est possible d'obtenir des compositions aqueuses ou hydroalcooliques facilement pulvérisables et/ou vaporisables.

La présente invention a donc pour objet une composition cosmétique comprenant, dans un milieu aqueux ou hydroalcoolique cosmétiquement acceptable, au moins un polymère fixant et/ou de conditionnement et au moins un copolymère acrylique formé de (a) environ 35 à 74% en poids d'un acrylate d'alkyle, (b) environ 25 à 65% en poids de méthacrylate d'alkyle et (c) environ 1 à 15% en poids d'un ou plusieurs acides carboxyliques éthyléniques ayant de 3 à 5 atomes de carbone, les radicaux alkyle ayant de 1 à 5 atomes de carbone et ces pourcentages étant exprimés en poids par rapport au poids total de copolymère.

L'invention a également pour objet l'utilisation du copolymère décrit ci-dessus pour améliorer la qualité de la vaporisation ou de la pulvérisation des compositions cosmétiques conditionnées par exemple en flacon pompe ou en aérosol et comprenant un polymère fixant et/ou de conditionnement.

Les sprays obtenus avec les compositions selon l'invention sont plus diffus, les gouttes sont plus fines. Les compositions ne moussent pas ou très peu.

Dans le cadre de la présente demande, on entend par compositions cosmétiques pour le maintien de la coiffure toute composition ayant pour fonction de fixer temporairement la forme de la coiffure, comme par exemple les laques, les compositions de mise en plis et sprays de coiffage. Par pouvoir fixant de la composition, on désigne l'aptitude de cette dernière à donner aux cheveux une cohésion de telle sorte que la mise en forme initiale de la coiffure soit conservée. Par polymère fixant, on entend tout polymère ayant pour fonction de fixer temporairement la forme de la coiffure.
Dans le cadre de la présente demande, on entend par polymère conditionneur tout polymère ayant pour fonction l'amélioration des propriétés cosmétiques des cheveux, en particulier la douceur, le démêlage, le toucher, l'électricité statique.

Selon l'invention, le copolymère acrylique décrit ci-dessus a généralement une température de transition vitreuse comprise entre 10 et 50°C environ et de préférence entre 20 et 40°C et encore plus particulièrement entre 25 et 35 °C. Ce copolymère peut présenter un poids moléculaire moyen en nombre compris entre 10000 et 50000, et de préférence entre 20000 et 40000.

L'acrylate d'alkyle est de préférence choisi parmi l'acrylate de méthyle, l'acrylate d'éthyle, l'acrylate de propyle et l'acrylate de butyle. L'acrylate d'éthyle est particulièrement préféré.
La concentration en acrylate d'alkyle est de préférence comprise entre 40 et 70% en poids et plus particulièrement entre 50 et 60% en poids par rapport au poids total du copolymère.

Le méthacrylate d'alkyle est de préférence choisi parmi le méthacrylate de méthyle, le méthacrylate d'éthyle, le méthacrylate de propyle et le méthacrylate de butyle. Le méthacrylate de méthyle est particulièrement préféré.
La concentration en méthacrylate d'alkyle est de préférence comprise entre 30 et 50% en poids et plus particulièrement entre 30 et 40% en poids par rapport au poids total du copolymère.

Les acides carboxyliques éthyléniques préférés sont l'acide acrylique, l'acide méthacrylique, l'acide crotonique, l'acide itaconique ou leurs mélanges. L'acide acrylique et l'acide méthacrylique sont particulièrement préférés. Selon l'invention, il est possible de mettre en oeuvre des sels de ces acides carboxyliques.

La concentration en acides carboxyliques éthyléniques, ou en leurs sels, est de préférence comprise entre 5 et 15% en poids et plus particulièrement entre 8 et 12% en poids par rapport au poids total du copolymère.

Dans un mode de réalisation particulièrement préféré de l'invention, l'acide acrylique est utilisé avec l'acide méthacrylique, chacun dans une concentration comprise entre 2 et 10% en poids, le total de ces deux acides n'excédant pas 15% en poids du poids total du copolymère.

Le copolymère peut également contenir des faibles quantités, c'est à dire moins de 10%, de préférence moins de 5% et plus particulièrement moins de 2%, d'un monomère polymérisable autre que ceux mentionnés ci-avant.

Le copolymère peut être utilisé sous forme d'une dispersion aqueuse. Généralement, la dispersion comprend alors au moins 0,05% de tensioactif permettant la mise en dispersion et le maintien en dispersion du polymère. Selon l'invention, on peut utiliser tout type de tensioactif dans ladite dispersion, mais de préférence un tensioactif non ionique, et plus particulièrement les alkyl(C₆-C₁₂)phénols polyoxyalkylénés.

La taille moyenne des particules du copolymère dans la dispersion est de préférence comprise entre 0,1 et 1 microns.

Selon un mode particulièrement préféré de mise en oeuvre de l'invention, on utilise un copolymère comprenant 50 à 60% en poids d'acrylate d'éthyle, 30 à 40% en poids de méthacrylate de méthyle, de 2 à 10% en poids d'acide acrylique, de 2 à 10% en poids d'acide méthacrylique, la concentration totale d'acide acrylique et méthacrylique n'excédant pas 15% en poids par rapport au poids total du copolymère acrylique.

Un tel copolymère est par exemple décrit dans la demande de brevet EP-A-590604 qui est ici incluse à titre de référence.

Une dispersion aqueuse du copolymère acrylique décrit ci-dessus comprenant 25% en poids de copolymère acrylate d'éthyle / méthacrylate de méthyle / acide méthacrylique / acide acrylique ayant une température de transition vitreuse d'environ 30°C est notamment vendu sous la dénomination AMERHOLD DR-25 par la société AMERCHOL.

Selon l'invention, les fonctions acide carboxylique du copolymère acrylique peuvent être neutralisées partiellement ou totalement.

Selon l'invention, on peut utiliser tout polymère fixant et/ou conditionneur connu en soi. On peut utiliser en particulier un polymère fixant et/ou conditionneur choisi parmi les polymères anioniques, cationiques, amphotères, non ioniques et leurs mélanges.
Les polymères conditionneurs sont de préférence choisis parmi les polymères cationiques, amphotères et leurs mélanges.
Les polymères fixants et/ou conditionneurs peuvent être utilisés sous forme solubilisée ou sous forme de latex (dispersion aqueuse de particules solides de polymère).

Ainsi, les polymères anioniques généralement utilisés sont des polymères comportant des groupements dérivés d'acide carboxylique, sulfonique ou phosphorique et ont un poids moléculaire moyen en nombre compris entre environ 500 et 5.000.000.
1) Les groupements carboxyliques sont apportés par des monomères mono ou diacides carboxyliques insaturés tels que ceux répondant à la formule : 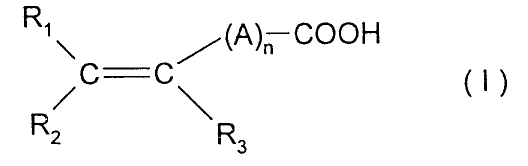 dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₁ désigne un atome d'hydrogène, un groupement phényle ou benzyle, R₂ désigne un atome d'hydrogène, un groupement alkyle inférieur ou carboxyle, R₃ désigne un atome d'hydrogène, un groupement alkyle inférieur, un groupement -CH₂ - COOH, phényle ou benzyle. Dans la formule précitée un radical alkyle inférieur désigne de préférence un groupement ayant 1 à 4 atomes de carbone et en particulier, méthyle et éthyle.
   Les polymères anioniques à groupements carboxyliques préférés selon l'invention sont :
   A) Les homo- ou copolymères d'acide acrylique ou méthacrylique ou leurs sels et en particulier les produits vendus sous les dénominations VERSICOL E ou K par la société ALLIED COLLOID, ULTRAHOLD par la société BASF, les copolymères d'acide acrylique et d'acrylamide vendus sous la forme de leur sel de sodium sous les dénominations RETEN 421, 423 ou 425 par la Société HERCULES, les sels de sodium des acides polyhydroxycarboxyliques.
   B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés. De tels polymères sont décrits en particulier dans le brevet français 1.222.944 et la demande allemande 2.330.956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀ par exemple de lauryle tel que celui vendu par la société ISP sous la dénomination ACRYLIDONE LM et les terpolymères acide méthacrylique/ acrylate d'éthyle/ acrylate de tertiobutyle tel que le produit vendu sous la dénomination LUVIMER 100 P par la société BASF.
   C) Les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ou encore un ester vinylique, allylique ou méthallylique d'un acide carboxylique α- ou β-cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 vendues par la société NATIONAL STARCH.
   D) Les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; ces polymères peuvent être estérifiés. De tels polymères sont décrits en particulier dans les brevets US 2.047.398, 2.723.248, 2.102.113, le brevet GB 839.805 et notamment ceux vendus sous les dénominations GANTREZ AN ou ES par la société ISP.
      Des polymères entrant également dans cette classe sont les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acrylique ou méthacrylique ou la vinylpyrrolidone dans leur chaîne, les fonctions anhydrides sont monoestérifiées ou monoamidifiées. Ces polymères sont par exemple décrits dans les brevets français 2.350.384 et 2.357.241 de la demanderesse.
   E) Les polyacrylamides comportant des groupements carboxylates.
2) Les polymères comprenant les groupements sulfoniques sont des polymères comportant des motifs vinylsulfonique, styrène sulfonique, naphtalène sulfonique ou acrylamido alkylsulfonique.

Ces polymères peuvent être notamment choisis parmi :
- les sels de l'acide polyvinylsulfonique ayant un poids moléculaire moyen en nombre compris entre environ 1.000 et 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone.
- les sels de l'acide polystyrène sulfonique les sels de sodium ayant un poids moléculaire moyen en nombre d'environ 500.000 et d'environ 100.000 vendus respectivement sous les dénominations Flexan 500 et Flexan 130 par National Starch. Ces composés sont décrits dans le brevet FR 2.198.719. - les sels d'acides polyacrylamide sulfoniques ceux mentionnés dans le brevet US 4.128.631 et plus particulièrement les sels de l'acide polyacrylamidoéthylpropane sulfonique.

Selon l'invention, les polymères anioniques sont de préférence choisis parmi les copolymères d'acide acrylique tels que les terpolymères acide acrylique/acrylate d'éthyle/N-tertiobutylacrylamide vendus par exemple sous la dénomination ULTRAHOLD STRONG par la société BASF, les copolymères dérivés d'acide crotonique tels que les terpolymères acétate de vinyle / tertio-butyl benzoate de vinyle / acide crotonique et les terpolymères acide crotonique/ acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique mono estérifiés vendus notamment sous la dénomination GANTREZ ES 425 par la société ISP, les copolymères d'acide méthacrylique et de méthacrylate de méthyle vendus sous la dénomination EUDRAGIT L par la société ROHM PHARMA, les copolymères d'acide méthacrylique et d'acrylate d'éthyle vendus sous la dénomination LUVIMER MAEX par la société BASF, les terpolymères vinylpyrrolidone/acide acrylique/méthacrylate de lauryle vendus sous la dénomination ACRYLIDONE LM par la société ISP, les terpolymères acide méthacrylique/ acrylate d'éthyle/ acrylate de tertiobutyle vendus sous la dénomination LUVIMER 100 P par la société BASF.

Les polymères cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant un poids moléculaire moyen en nombre compris entre 500 et environ 5.000.000 et de préférence entre 1000 et 3.000.000.

Dans le cadre des polymères cationiques fixants, on préfère les polymères cationiques de conductivité inférieure ou égale à 1 mohm⁻¹cm⁻¹ et dont la viscosité à 1 % dans l'eau est inférieure à 20 cps ( 20mPa.s). La viscosité est mesurée à l'aide d'un Rheomat RM 180 (Contraves TV mobile 1) de Mettler.

Parmi ces polymères, on peut citer plus particulièrement les polymères cationiques suivants:
(1) les homopolymères ou copolymères dérivés d'esters ou d'amides acryliques ou méthacryliques et comportant au moins un des motifs de formules suivantes: 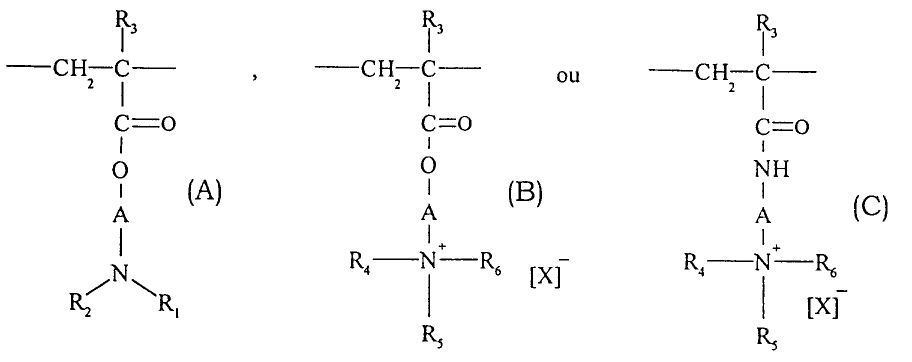 dans lesquelles:
   R₁ et R₂ représentent hydrogène ou un groupe alkyle ayant de 1 à 6 atomes de carbone ;
   R₃ désigne H ou CH₃;
   A est un groupe alkyle linéaire ou ramifié de 1 à 6 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle ;
   X désigne un anion méthosulfate ou un halogénure tel que chlorure ou bromure.

   Les copolymères de la famille (1) contiennent en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs, des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisés au sulfate de diméthyle vendus sous la dénomination HERCOFLOC par la société HERCULES,
   - les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
   - les copolymère d'acrylamide et de méthosulfate de méthacryloyloxyéthyltriméthylammonium vendus sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tels que le produit vendu sous la dénomination GAFFIX VC 713 par la société ISP,
   - et les copolymères vinylpyrrolidone / méthacrylamide de diméthylaminopropyle quaternisés tels que le produit vendu sous la dénomination "GAFQUAT HS 100" par la société ISP.
(2) les polysaccharides quaternisés décrits plus particulièrement dans les brevets américains 3.589.578 et 4.031.307 tel que le produit commercialisé sous la dénomination JAGUAR C 13 S par la société MEYHALL.
(3) les copolymères quaternaires de vinylpyrrolidone et de vinylimidazole.
(4) les chitosanes ou leurs sels.
   les sels utilisables sont en particulier l'acétate, le lactate, le glutamate, le gluconate ou le pyrrolidone carboxylate de chitosane.
   Parmi ces composés, on peut citer le chitosane ayant un taux de désacétylation de 90,5% vendu sous la dénomination KYTAN BRUT STANDARD par la société ABER TECHNOLOGIES, le pyrrolidone carboxylate de chitosane vendu sous la dénomination KYTAMER PC par la société AMERCHOL.

Les polymères amphotères utilisables conformément à l'invention peuvent être choisis parmi les polymères comportant des motifs A et B répartis statistiquement dans la chaîne polymère où A désigne un motif dérivant d'un monomère comportant au moins un atome d'azote basique et B désigne un motif dérivant d'un monomère acide comportant un ou plusieurs groupements carboxyliques ou sulfoniques ou bien A et B peuvent désigner des groupements dérivant de monomères zwittérioniques de carboxybétaïnes ou de sulfobétaïnes; A et B peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ou bien A et B font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537.
(2) les polymères dérivés de diallyldialkylammonium et d'au moins un monomère anionique tels que les polymères comportant environ 60 à environ 99% en poids d'unités dérivées d'un monomère de diallyldialkylammonium quaternaire dans lequel les groupements alkyle sont choisis indépendamment parmi les groupements alkyle ayant 1 à 18 atomes de carbone et dans lequel l'anion est dérivé d'un acide ayant une constante d'ionisation supérieure à 10⁻¹³ et 1 à 40% en poids de ce polymère, d'un monomère anionique choisi parmi les acides acrylique ou méthacrylique, le poids moléculaire moyen en nombre de ce polymère étant compris entre environ 50.000 et 10.000.000 déterminé par chromatographie par perméation de gel. De tels polymères sont décrits dans la demande EP-A-269.243.
   Les polymères préférés sont entre autres les polymères comportant des groupements alkyle choisis parmi les groupements ayant 1 à 4 atomes de carbone et plus particulièrement des groupements méthyle, éthyle.
   Parmi ces polymères, les copolymères de chlorure de diméthyldiallylammonium ou de diéthyldiallylammonium et d'acide acrylique sont particulièrement préférés.
   Ces polymères sont par exemple vendus sous les dénominations "MERQUAT 280" et "MERQUAT 295" par la société MERCK.
   On peut également utiliser les terpolymères de chlorure de diméthyldiallylammonium/ acide acrylique / acrylamide vendu sous la dénomination "MERQUAT PLUS 3330" par la société MERCK.
(3) les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
      Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl acrylamide, le N-tertiooctyl acrylamide, le N-octylacrylamide, le N-décylacrylamide, le N-dodécylacrylamide ainsi que les méthacrylamides correspondants.
      Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.

   Les comonomères basiques préférés sont des méthacrylates d'aminoéthyle, de butyl aminoéthyle, de N,N'-diméthylaminoéthyle, de N-tertio-butylaminoéthyle. On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(4) les polyamino amides réticulés et alcoylés partiellement ou totalement dérivant de polyaminoamides de formule générale :

   ⁅CO―R₁₀―CO―Z⁆ (II)

   dans laquelle R₁₀ représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de la réaction d'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical

      ―NH⁅(CH₂)ₓ―NH⁆ₙ (III)

      où x=2 et n=2 ou 3 ou bien x=3 et n=2 ce radical dérivant de la diéthylène triamine, de la triéthylène tétraamine ou de la dipropylène triamine;
   b) dans les proportions de 0 à 40 moles % le radical (III) ci-dessus, dans lequel x=2 et n=1 et qui dérive de l'éthylènediamine, ou le radical dérivant de la pipérazine : 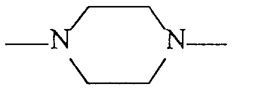
   c) dans les proportions de 0 à 20 moles % le radical -NH-(CH₂)₆-NH-dérivant de l'hexaméthylènediamine, ces polyaminoamides étant réticulés par réaction d'addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
      Les acides carboxyliques saturés sont choisis de préférence parmi les acides ayant 6 à 10 atomes de carbone tels que l'acide adipique, triméthyl-2,2,4- et -2,4,4-adipique, téréphtalique, les acides à double liaison éthylénique sont par exemple les acides acrylique, méthacrylique, itaconique.
      Les alcanes sultones utilisées dans l'alcoylation sont de préférence la propane ou la butane sultone, les sels des agents d'alcoylation sont de préférence les sels de sodium ou de potassium.
(5) les polymères comportant des motifs zwittérioniques de formule : 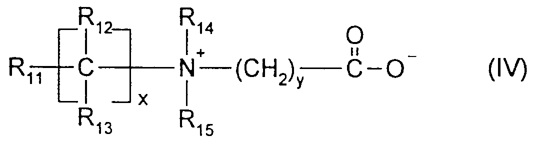 dans laquelle R₁₁ désigne un groupement insaturé polymérisable tel qu'un groupement acrylate, méthacrylate, acrylamide ou méthacrylamide, x et y représente un nombre entier de 1 à 3, R₁₂ et R₁₃ représentent hydrogène, méthyle, éthyle ou propyle, R₁₄ et R₁₅ représentent un atome d'hydrogène ou un radical alkyle de telle façon que la somme des atomes de carbone dans R₁₄ et R₁₅ ne dépasse pas 10.
   Les polymères comprenant de telles unités peuvent également comporter des motifs dérivés de monomères non zwittérioniques tels que la vinylpyrrolidone, l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymére de méthacrylate de méthyle / diméthyl carboxyméthylammonio éthylméthacrylate de méthyle tel que le produit vendu sous la dénomination DIAFORMER par la société SANDOZ.
(6) les polymères dérivés du chitosane comportant des motifs monomères répondant aux formules suivantes : 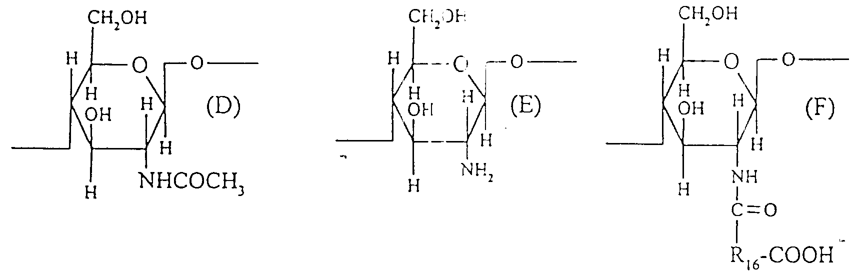 le motif D étant présent dans des proportions comprises entre 0 et 30%, le motif E dans des proportions comprises entre 5 et 50% et le motif F dans des proportions comprises entre 30 et 90%, étant entendu que dans ce motif F, R₁₆ représente un radical de formule : 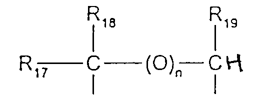 dans laquelle si n=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₁₇, R₁₈ et R₁₉ étant dans ce cas un atome d'hydrogène ;
   ou si n=1, R₁₇, R₁₈ et R₁₉ représentent chacun un atome d'hydrogène, ainsi que les sels formés par ces composés avec des bases ou des acides.
(7) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(8) Les polymères répondant à la formule générale (V) décrits dans le brevet français 1 400 366 : 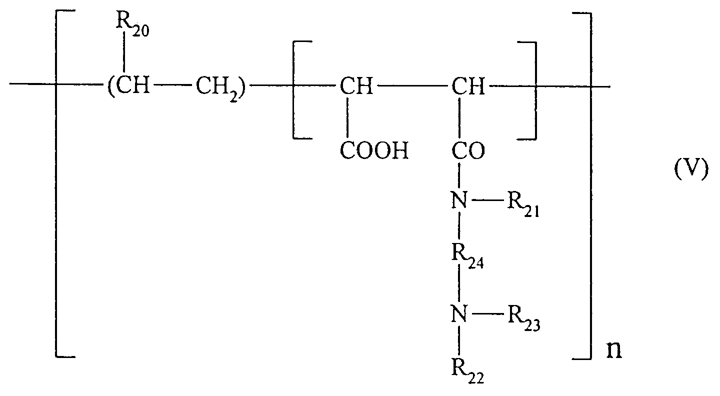 dans laquelle R₂₀ représente un atome d'hydrogène, un radical CH₃O, CH₃CH₂O, phényle, R₂₁ désigne l'hydrogène ou un radical alkyle inférieur tel que méthyle, éthyle, R₂₂ désigne l'hydrogène ou un radical alkyle inférieur tel que méthyle, éthyle, R₂₃ désigne un radical alkyle inférieur tel que méthyle, éthyle ou un radical répondant à la formule : -R₂₄-N(R₂₂)₂, R₂₄ représentant un groupement -CH₂-CH₂- , -CH₂-CH₂-CH₂- , -CH₂-CH(CH₃)- , R₂₂ ayant les significations mentionnées ci-dessus,
   ainsi que les homologues supérieurs de ces radicaux et contenant jusqu'à 6 atomes de carbone.
(9) Des polymères amphotères du type -A-Z-A-Z choisis parmi:
   a) les polymères obtenus par action de l'acide chloracétique ou le chloracétate de sodium sur les composés comportant au moins un motif de formule :

      -A-Z-A-Z-A- (VI)

      où A désigne un radical 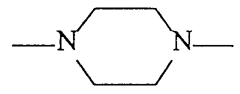 et Z désigne le symbole B ou B', B ou B' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne.
   b) Les polymères de formule :

      -A-Z-A-Z- (VI')

      où A désigne un radical 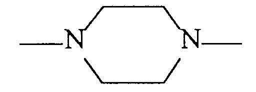 et Z désigne le symbole B ou B' et au moins une fois B'; B ayant la signification indiquée ci-dessus et B' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(10) les copolymères alkyl(C₁-C₅)vinylèther / anhydride maléique modifié partiellement par semiamidification avec une N,N-dialkylaminoalkylamine telle que la N,N-diméthylaminopropylamine ou par semiestérification avec une N,N-dialkyl aminoalcanol. Ces copolymères peuvent également comporter d'autres comonomères vinyliques tels que le vinylcaprolactame.

Les polymères amphotères particulièrement préférés selon l'invention sont ceux de la famille (3) tels que les copolymères dont la dénomination CTFA est Octylacrylamide/acrylates/butylaminoethylmethacrylate copolymer tels que les produits vendus sous la dénomination AMPHOMER ou LOVOCRYL 47 par la société NATIONAL STARCH.

Le ou les polymères fixants et/ou conditionneurs sont par exemple présents dans des concentrations comprises entre 0,1% et 20% en poids, et de préférence dans des concentrations comprises entre 1% et 10% en poids, par rapport au poids total de la composition.

La concentration en copolymère acrylique est en général comprise entre 0,01 et 15% en poids par rapport au poids total de la composition et de préférence entre 0,05 et 8% en poids.

Le rapport en poids entre le polymère fixant et/ou conditionneur et le copolymère acrylique peut être compris entre 0,1 et 10 et de préférence entre 0,5 et 5.

Le milieu cosmétiquement acceptable est de préférence constitué par de l'eau ou un mélange d'eau et de solvants cosmétiquement acceptables tels que des monoalcools, des polyalcools, des éthers de glycol, qui peuvent être utilisés seuls ou en mélange.

On peut citer plus particulièrement les alcools inférieurs tels que l'éthanol, l'isopropanol, les polyalcools tels que le diéthylèneglycol, les éthers de glycol tels que les alkyléthers de glycol ou de diéthylèneglycol.

La concentration en solvants est en général comprise entre 0 et 80% en poids et de préférence entre 0 et 55% en poids par rapport au poids total de la composition.

De préférence, le milieu cosmétiquement acceptable est essentiellement constitué d'eau.

Le pH des compositions selon l'invention est généralement compris entre 2 et 9, et en particulier entre 3 et 8. Il peut être ajusté à la valeur choisie au moyen d'agents alcalinisants ou acidifiants habituellement utilisés en cosmétique pour ce type d'application.

Lorsque la composition selon l'invention est pressurisée sous forme d'aérosol en vue d'obtenir une laque, elle comprend au moins un agent propulseur qui peut être choisi parmi les hydrocarbures volatils tels que le n-butane, le propane, l'isobutane, le pentane, les hydrocarbures chlorés et/ou fluorés et leurs mélanges. On peut également utiliser en tant qu'agent propulseur le gaz carbonique, le protoxyde d'azote, le diméthyléther, l'azote, l'air comprimé et leurs mélanges.

La concentration en propulseur est généralement comprise entre 10 et 50% en poids par rapport au poids total de la composition pressurisée et de préférence entre 15 et 35% en poids.

Les compositions selon l'invention pressurisée ou non peuvent encore contenir des agents tensioactifs, des agents conservateurs, des séquestrants, des adoucissants, des colorants, des agents modificateurs de viscosité, des agents modificateurs de mousse, des agents anti-mousse, des agents nacrants, des agents hydratants, des agents antipelliculaires, des agents antiséborrhéiques, des filtres solaires, des protéines, des vitamines, des plastifiants, des hydroxyacides, des électrolytes et des parfums.

Les compositions selon l'invention peuvent également contenir d'autres agents conditionneurs. Ceux-ci peuvent alors être choisis parmi les huiles et les cires naturelles ou synthétiques, les alcools gras, les esters d'alcools polyhydriques, les glycérides, les gommes et résines de silicone ou les mélanges de ces différents composés.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions utilisées selon l'invention sont par exemple des compositions capillaires rincées ou non rincées.

Elles sont plus particulièrement des lotions de mise en plis, des lotions pour le brushing, des compositions de fixation (laques) et de coiffage. Les lotions peuvent être conditionnées sous diverses formes notamment dans des vaporisateurs, des flacons pompe ou dans des récipients aérosols afin d'assurer une application de la composition sous forme vaporisée.

L'invention a encore pour objet un procédé de traitement cosmétique des matières kératiniques, telles que les cheveux, caractérisé en ce qu'il consiste à appliquer sur matières kératiniques une composition cosmétique telle que définie précédemment, puis à effectuer éventuellement un rinçage à l'eau, après un éventuel temps de pose.

Des exemples concrets mais nullement limitatifs vont maintenant illustrer l'invention.

Dans les exemples, MA signife matière active.

### EXEMPLE 1

On a préparé deux compositions selon l'invention A et B et on les a comparé aux compositions C et D contenant chacune un seul des deux polymères. Les quatre compositions ont été conditionnées dans un flacon-pompe. Un panel de 5 testeurs expérimentés a évalué l'aspect du spray et la présence ou l'absence de mousse à la sortie du récipient ou sur les cheveux.

Pour l'évaluation de l'aspect du spray, la notation allait de 0 (mauvais) signifiant que le spray n'était pas diffus, que les gouttelettes pulvérisées n'étaient pas fines à 5 (excellent) qui signifiait que le spray était bien diffus et que les gouttelettes pulvérisées étaient très fines.
Pour l'évaluation de la présence ou de l'absence de mousse à la sortie du récipient ou sur les cheveux, la notation allait de 0 (mauvais) signifiant qu'il y avait une mousse abondante inacceptable à 5 (excellent) qui signifiait qu'il n'y avait pas du tout de mousse.

Les résultats sont rassemblés dans le tableau ci-dessous :

| En gMA | **A (Invention)** | **B (Invention)** | **C** | **D** |
|---|---|---|---|---|
| **ULTRAHOLD STRONG**⁽¹⁾ | 6 | 4 | 8 | - |
| **AMERHOLD DR 25**⁽²⁾ | 2 (en copolymère) | 4 (en copolymère) | - | 8 (en copolymère) |
| **AMP**⁽³⁾ **qs** | pH 9 | pH 9 | pH 9 | pH 9 |
| **Eau qsp** | 100 | 100 | 100 | 100 |
| **Aspect du spray** | 4 | 5 | 1 | 3 |
| **Absence ou présence de mousse** | 4 | 5 | 1 | 4,5 |

| | | | | |
|---|---|---|---|---|
| ⁽¹⁾ULTRAHOLD STRONG de BASF : terpolymère acide acrylique/acrylate d'éthyle/N-tertiobutylacrylamide. | | | | |
| ⁽²⁾AMERHOLD DR 25 de AMERCHOL : copolymère acrylate d'éthyle / méthacrylate de méthyle / acide méthacrylique / acide acrylique ayant une température de transition vitreuse d'environ 30°C en dispersion aqueuse comprenant 25% en poids du copolymère acrylique. | | | | |
| ⁽³⁾AMP : Amino-2 méthyl-2 propanol | | | | |

Les compositions selon l'invention A et B ont un spray diffus et il n'y a pas de mousse à la sortie du flacon-pompe ou sur les cheveux.

### EXEMPLE 2

On a préparé deux compositions selon l'invention E et F et on les a comparé aux compositions G et H contenant chacune un seul des deux polymères. Les quatre compositions ont été conditionnées dans un flacon-pompe. Un panel de 5 testeurs expérimentés a évalué l'aspect du spray et la présence ou l'absence de mousse à la sortie du récipient ou sur les cheveux.

Pour l'évaluation de l'aspect du spray, la notation allait de 0 (mauvais) signifiant que le spray n'était pas diffus, que les gouttelettes pulvérisées n'étaient pas fines à 5 (excellent) qui signifiait que le spray était bien diffus et que les gouttelettes pulvérisées étaient très fines.
Pour l'évaluation de la présence ou de l'absence de mousse à la sortie du récipient ou sur les cheveux, la notation allait de 0 (mauvais) signifiant qu'il y avait une mousse abondante inacceptable à 5 (excellent) qui signifiait qu'il n'y avait pas du tout de mousse.

Les résultats sont rassemblés dans le tableau ci-dessous :

| En gMA | **E (Invention)** | **F (Invention)** | **G** | **H** |
|---|---|---|---|---|
| **LOVOCRYL 47**⁽¹⁾ | 6 | 4 | 8 | - |
| **AMERHOLD DR 25**⁽²⁾ | 2 (en copolymère) | 4 (en copolymère) | - | 8 (en copolymère) |
| **AMP**⁽³⁾ **qs pH** | 9 | 9 | 9 | 9 |
| **Eau qsp** | 100 | 100 | 100 | 100 |
| **Aspect du spray** | 5 | 5 | 2 | 3 |
| **Absence ou présence de mousse** | 5 | 5 | 3 | 4,5 |

| | | | | |
|---|---|---|---|---|
| ⁽¹⁾LOVOCRYL 47 de NATIONAL STARCH : Octylacrylamide/acrylates/butylaminoethylmethacrylate copolymer (CTFA). | | | | |
| ⁽²⁾AMERHOLD DR 25 de AMERCHOL : copolymère acrylate d'éthyle / méthacrylate de méthyle / acide méthacrylique / acide acrylique ayant une température de transition vitreuse d'environ 30°C en dispersion aqueuse comprenant 25% en poids du copolymère. | | | | |
| ⁽³⁾AMP : Amino-2 méthyl-2 propanol | | | | |

Les compositions selon l'invention E et F ont un spray diffus et il n'y a pas de mousse à la sortie du flacon-pompe ou sur les cheveux.

### EXEMPLE 3

On a préparé une composition J selon l'invention et on l'a comparé aux compositions K et L contenant chacune un seul des deux polymères. Les trois compositions ont été conditionnées en aérosol. Un panel de 5 testeurs expérimentés a évalué l'aspect du spray et l'absence ou la présence de mousse à la sortie du récipient ou sur les cheveux.

Pour l'évaluation de l'aspect du spray, la notation allait de 0 (mauvais) signifiant que le spray n'était pas diffus, que les gouttelettes pulvérisées n'étaient pas fines à 5 (excellent) qui signifiait que le spray était bien diffus et que les gouttelettes pulvérisées étaient très fines.
Pour l'évaluation de la présence ou de l'absence de mousse à la sortie du récipient ou sur les cheveux, la notation allait de 0 (mauvais) signifiant qu'il y avait une mousse abondante inacceptable à 5 (excellent) qui signifiait qu'il n'y avait pas du tout de mousse.

Les résultats sont rassemblés dans le tableau ci-dessous :

| En gMA | **J (Invention)** | **K** | **L** |
|---|---|---|---|
| **ULTRAHOLD STRONG**⁽¹⁾ | 2 | 4 | - |
| **AMERHOLD DR 25**⁽²⁾ | 2 (en copolymère) | - | 4 (en copolymère) |
| **AMP**⁽³⁾ **qs pH** | 7 | 7 | 7 |
| **Ethanol** | 20 | 20 | 20 |
| **Eau** | 41 | 41 | 41 |
| **DME**⁽⁴⁾ | 35 | 35 | 35 |
| **Aspect du spray** | 5 | 2 | 4 |
| **Absence ou présence de mousse** | 5 | 1 | 4 |

| | | | |
|---|---|---|---|
| ⁽¹⁾ULTRAHOLD STRONG de BASF : terpolymère acide acrylique/acrylate d'éthyle/N-tertiobutylacrylamide. | | | |
| ⁽²⁾AMERHOLD DR 25 de AMERCHOL : copolymère acrylate d'éthyle / méthacrylate de méthyle / acide méthacrylique / acide acrylique ayant une température de transition vitreuse d'environ 30°C en dispersion aqueuse comprenant 25% en poids du copolymère. | | | |
| ⁽³⁾AMP : Amino-2 méthyl-2 propanol | | | |
| ⁽⁴⁾DME : Diméthyléther (propulseur) | | | |

La composition selon l'invention J a un spray diffus et il n'y a pas de mousse à la sortie de l'aérosol ou sur les cheveux.

### EXEMPLE 4

On donne ici un exemple concret d'une composition selon l'invention pressurisée en aérosol de composition suivante :

| | |
|---|---|
| - Octylacrylamide/acrylates/butylaminoethylmethacrylate copolymer (LOVOCRYL 47 de NATIONAL STARCH) | 2 gMA |
| - copolymère acrylate d'éthyle / méthacrylate de méthyle / acide méthacrylique / acide acrylique vendu en dispersion aqueuse à 25% de MA sous la dénomination AMERHOLD DR 25 par AMERCHOL | 3 gMA |
| - Amino-2 méthyl-2 propanol-1 qs | pH 9 |
| - Ethanol | 20 g |
| - Diméthyléther | 35 g |
| - Eau qsp | 100g |

### EXEMPLE 5

On donne ici un exemple concret d'une composition selon l'invention conditionnée en flacon-pompe de composition suivante :

| | |
|---|---|
| - Terpolymère méthacrylate de diméthyl amino éthyle/vinylcaprolactame/vinylpyrrolidone en solution dans l'éthanol à 37% de MA (GAFFIX VC 713 de ISP) | 2 gMA |
| - Copolymère acrylate d'éthyle / méthacrylate de méthyle / acide méthacrylique / acide acrylique vendu en dispersion aqueuse à 25% de MA sous la dénomination AMERHOLD DR 25 par AMERCHOL | 2 gMA |
| - Amino-2 méthyl-2 propanol-1 qs | pH 7 |
| -Eau qsp | 100 g |

### EXEMPLE 6

On donne ici un exemple concret d'une composition selon l'invention conditionnée en flacon-pompe de composition suivante :

| | |
|---|---|
| - Copolymère acide méthacrylique/méthacrylate d'hydroxyéthyl/acrylate acrylate de butyle/méthacrylate de méthyle vendu en dispersion aqueuse à 41% de matière active sous la dénomination ACUDYNE 255 par la société SEPPIC | 4 gMA |
| - Copolymère acrylate d'éthyle / méthacrylate de méthyle / acide acide méthacrylique / acide acrylique vendu en dispersion aqueuse à 25% de MA sous la dénomination AMERHOLD DR 25 par AMERCHOL | 2 gMA |
| - Amino-2 méthyl-2 propanol-1 qs | pH 7 |
| -Eau qsp | 100 g |

## Revendications

1. Composition cosmétique, caractérisée par le fait qu'elle comprend dans un milieu aqueux ou hydroalcoolique cosmétiquement acceptable, au moins un polymère fixant et/ou conditionneur et au moins un copolymère acrylique formé de (a) environ 35 à 74% en poids d'un acrylate d'alkyle, (b) environ 25 à 65% de méthacrylate d'alkyle et (c) environ 1 à 15% d'un ou plusieurs acides carboxyliques éthyléniques ayant de 3 à 5 atomes de carbone , les radicaux alkyle ayant de 1 à 5 atomes de carbone et les pourcentages étant exprimés par rapport au poids total du copolymère.

2. Composition selon la revendication 1, caractérisée en ce que le copolymère acrylique présente une température de transition vitreuse comprise entre 10 et 50°C environ, un poids moléculaire moyen en nombre compris entre 10000 et 50000.

3. Composition selon l'une quelconque des revendications 1 et 2, caractérisée en ce que le copolymère acrylique est un copolymère acrylate d'éthyle / méthacrylate de méthyle / acide méthacrylique / acide acrylique.

4. Composition selon la revendication précédente, caractérisée en ce que le copolymère acrylique comprend 50 à 60% en poids d'acrylate d'éthyle, 30 à 40% en poids de méthacrylate de méthyle, de 2 à 10% en poids d'acide acrylique, de 2 à 10% en poids d'acide méthacrylique, la concentration totale d'acide acrylique et méthacrylique n'excédant pas 15% en poids par rapport au poids total du copolymère.

5. Composition selon l'une quelconque des revendications précédentes, caractérisée en ce que le polymère fixant et/ou conditionneur est présent dans des concentrations comprises entre 0,1% et 20% en poids par rapport au poids total de la composition.

6. Composition selon la revendication 5, caractérisée en ce que le polymère fixant et/ou conditionneur est présent dans des concentrations comprises entre 1% et 10% en poids, par rapport au poids total de la composition.

7. Composition selon l'une quelconque des revendications précédentes caractérisée en ce que la concentration en copolymère acrylique est comprise entre 0,01 et 15% en poids par rapport au poids total de la composition.

8. Composition selon la revendication 7, caractérisée en ce la concentration en copolymère acrylique est comprise entre 0,05 et 8% en poids par rapport au poids total de la composition.

9. Composition selon l'une quelconque des revendications précédentes caractérisée en ce que le polymère fixant et/ou conditionneur est choisi parmi les polymères anioniques, cationiques, amphotères, non ioniques et leurs mélanges.

10. Composition selon la revendication précédente, caractérisée en ce que le polymère anionique est choisi parmi :
- les copolymères d'acide acrylique tels que les terpolymères acide acrylique/acrylate d'éthyle/N-tertiobutylacrylamide ;
- les copolymères dérivés d'acide crotonique tels que les terpolymères acétate de vinyle / tertio-butyl benzoate de vinyle / acide crotonique et les terpolymères acide crotonique/acétate de vinyle/néododécanoate de vinyle ;
- les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique mono estérifié.
- les copolymères d'acide méthacrylique et de méthacrylate de méthyle;
- les copolymères d'acide méthacrylique et d'acrylate d'éthyle;
- les terpolymères vinylpyrrolidone/acide acrylique/méthacrylate de lauryle ;
- les terpolymères acide méthacrylique/ acrylate d'éthyle/ acrylate de tertiobutyle.

11. Composition selon la revendication 9, caractérisée en ce que le polymère cationique est choisi parmi :
- les copolymères d'acrylamide et de diméthylaminoéthyl méthacrylate quaternisés au sulfate de diméthyle,
- les copolymères d'acrylamide et de chlorure de méthacryloyloxyéthyltriméthylammonium,
- les copolymères d'acrylamide et de méthosulfate de méthacryloyloxyéthyltriméthylammonium,
- les copolymères vinylpyrrolidone / acrylate ou méthacrylate de dialkylaminoalkyle quaternisés ou non,
- les terpolymères méthacrylate de diméthyl amino éthyle/ vinylcaprolactame/ vinylpyrrolidone,
- et les copolymère vinylpyrrolidone / méthacrylamide de diméthylaminopropyle quaternisés.

12. Composition selon la revendication 9, caractérisée en ce que le polymère amphotère est choisi parmi les polymères comportant des motifs dérivant:
a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

13. Composition selon la revendication 12, caractérisée en ce que le polymère amphotère est choisi parmi les copolymères dont la dénomination CTFA est Octylacrylamide/ acrylates/ butylaminoethylmethacrylate copolymer.

14. Composition selon l'une quelconque des revendications précédentes, caractérisée par le fait qu'elle est pressurisée sous la forme d'un aérosol.

15. Composition selon la revendication 14, caractérisée par le fait qu'elle comprend de 10 à 50 % en poids d'un propulseur par rapport au poids total de la composition.

16. Utilisation d'un copolymère acrylique tel que défini dans l'une quelconque des revendications 1 à 4 pour améliorer la qualité de la vaporisation et/ou la pulvérisation des compositions cosmétiques comprenant un polymère fixant et/ou conditionneur.

17. Utilisation d'un copolymère acrylique décrit dans l'une quelconque des revendications 1 à 4 pour améliorer la qualité de la vaporisation et/ou la pulvérisation des compositions cosmétiques pressurisées en aérosol comprenant un polymère fixant et/ou conditionneur.

18. Procédé de traitement cosmétique des matières kératiniques, telles que les cheveux, caractérisé en ce qu'il consiste à appliquer sur les matières kératiniques une composition cosmétique telle que définie dans l'une quelconque des revendications 1 à 15, puis à effectuer éventuellement un rinçage à l'eau, après un éventuel temps de pose.

## Claims

1. Cosmetic composition, characterized in that it comprises, in a cosmetically acceptable aqueous or aqueous/alcoholic medium, at least one fixing and/or conditioning polymer and at least one acrylic copolymer formed from (a) approximately 35 to 74 % by weight of an alkyl acrylate, (b) approximately 25 to 65 % of alkyl methacrylate and (c) approximately 1 to 15 % of one or a number of ethylenic carboxylic acids having from 3 to 5 carbon atoms, the alkyl radicals having from 1 to 5 carbon atoms and the percentages being expressed with respect to the total weight of the copolymer.

2. Composition according to Claim 1, characterized in that the acrylic copolymer exhibits a glass transition temperature of between 10 and 50°C approximately and a number-average molecular weight of between 10,000 and 50,000.

3. Composition according to either one of Claims 1 and 2, characterized in that the acrylic copolymer is an ethyl acrylate/methyl methacrylate/methacrylic acid/acrylic acid copolymer.

4. Composition according to the preceding claim, characterized in that the acrylic copolymer comprises 50 to 60 % by weight of ethyl acrylate, 30 to 40 % by weight of methyl methacrylate, from 2 to 10 % by weight of acrylic acid and from 2 to 10 % by weight of methacrylic acid, the total concentration of acrylic and methacrylic acid not exceeding 15 % by weight with respect to the total weight of the copolymer.

5. Composition according to any one of the preceding claims, characterized in that the fixing and/or conditioning polymer is present in concentrations of between 0.1 % and 20 % by weight with respect to the total weight of the composition.

6. Composition according to Claim 5, characterized in that the fixing and/or conditioning polymer is present in concentrations of between 1 % and 10 % by weight with respect to the total weight of the composition.

7. Composition according to any one of the preceding claims, characterized in that the concentration of acrylic copolymer is between 0.01 and 15 % by weight with respect to the total weight of the composition.

8. Composition according to Claim 7, characterized in that the concentration of acrylic copolymer is between 0.05 and 8 % by weight with respect to the total weight of the composition.

9. Composition according to any one of the preceding claims, characterized in that the fixing and/or conditioning polymer is chosen from anionic, cationic, amphoteric and non-ionic polymers and their mixtures.

10. Composition according to the preceding claim, characterized in that the anionic polymer is chosen from:
- copolymers of acrylic acid, such as the acrylic acid/ethyl acrylate/N-tert-butylacrylamide terpolymers;
- copolymers derived from crotonic acid, such as vinyl acetate/vinyl tert-butylbenzoate/crotonic acid terpolymers and crotonic acid/vinyl acetate/vinyl neododecanoate terpolymers;
- polymers derived from maleic, fumaric or itaconic acids or anhydrides with vinyl esters, vinyl ethers, vinyl halides, phenylvinyl derivatives or acrylic acid and its esters, such as monoesterified maleic anhydride/methyl vinyl ether copolymers;
- copolymers of methacrylic acid and of methyl methacrylate;
- copolymers of methacrylic acid and of ethyl acrylate;
- vinylpyrrolidone/acrylic acid/lauryl methacrylate terpolymers;
- methacrylic acid/ethyl acrylate/tert-butyl acrylate terpolymers.

11. Composition according to Claim 9, characterized in that the cationic polymer is chosen from:
- copolymers of acrylamide and of dimethylaminoethyl methacrylate, quaternized with dimethyl sulphate,
- copolymers of acrylamide and of methacryloyloxyethyltrimethylammonium chloride,
- copolymers of acrylamide and of methacryloyloxyethyltrimethylammonium methyl sulphate,
- optionally quaternized vinylpyrrolidone/dialkylaminoalkyl acrylate or methacrylate copolymers,
- dimethylaminoethyl methacrylate/vinylcaprolactam/vinylpyrrolidone terpolymers,
- and quaternized dimethylaminopropylmethacrylamide/vinylpyrrolidone copolymers.

12. Composition according to Claim 9, characterized in that the amphoteric polymer is chosen from polymers containing units deriving:
a) from at least one monomer chosen from acrylamides or methacrylamides substituted on the nitrogen by an alkyl radical,
b) from at least one acidic comonomer containing one or a number of reactive carboxyl groups, and
c) from at least one basic comonomer, such as esters containing primary, secondary, tertiary and quaternary amine substituents of acrylic and methacrylic acids and the product from the quaternization of dimethylaminoethyl methacrylate with dimethyl or diethyl sulphate.

13. Composition according to Claim 12, characterized in that the amphoteric polymer is chosen from copolymers whose CTFA name is Octylacrylamide/acrylates/butylaminoethyl methacrylate copolymer.

14. Composition according to any one of the preceding claims, characterized in that it is pressurized in the form of an aerosol.

15. Composition according to Claim 14, characterized in that it comprises from 10 to 50 % by weight of a propellant with respect to the total weight of the composition.

16. Use of an acrylic copolymer as defined in any one of Claims 1 to 4 for improving the quality of the vaporization and/or the spraying of cosmetic compositions comprising a fixing and/or conditioning polymer.

17. Use of an acrylic copolymer described in any one of Claims 1 to 4 for improving the quality of the vaporization and/or the spraying of aerosol-pressurized cosmetic compositions comprising a fixing and/or conditioning polymer.

18. Process for the cosmetic treatment of keratinous substances, such as hair, characterized in that it consists in applying a cosmetic composition as defined in any one of Claims 1 to 15 to the keratinous substances and in then optionally rinsing with water, after an optional setting time.

## Patentansprüche

1. Kosmetische Zusammensetzung, dadurch gekennzeichnet, daß sie in einem kosmetisch akzeptablen wässerigen oder wässerig-alkoholischen Medium mindestens ein fixierendes und/oder konditionierendes Polymer und mindestens ein Acrylcopolymer, das aus (a) ungefähr 35 bis 74 Gew.-% eines Alkylacrylats, (b) ungefähr 25 bis 65 Gew.-% Alkylmethacrylat und (c) ungefähr 1 bis 15 Gew.-% einer oder mehrerer ethylenisch ungesättigter Carbonsäuren mit 3 bis 5 Kohlenstoffatomen gebildet wird, enthält, wobei die Alkylgruppen 1 bis 5 Kohlenstoffatome aufweisen und die Prozentangaben auf das Gesamtgewicht des Copolymeren bezogen sind.

2. Zusammensetzung nach Anspruch 1, dadurch gekennzeichnet, daß das Acrylcopolymer eine Glasübergangstemperatur im Bereich von ungefähr 10 bis 50 °C und ein Zahlenmittel des Molekulargewichts im Bereich von 10 000 bis 50 000 aufweist.

3. Zusammensetzung nach Anspruch 1 oder 2, dadurch gekennzeichnet, daß das Acrylcopolymer ein Ethylacrylat-Methylmethacrylat-Methacrylsäure-Acrylsäure-Copolymer ist.

4. Zusammensetzung nach Anspruch 3, dadurch gekennzeichnet, daß das Acrylcopolymer 50 bis 60 Gew.-% Ethylacrylat, 30 bis 40 Gew.-% Methylmethacrylat, 2 bis 10 Gew.-% Acrylsäure und 2 bis 10 Gew.-% Methacrylsäure enthält, wobei die Gesamtkonzentration an Acrylsäure und Methacrylsäure 15 Gew.-%, bezogen auf das Gesamtgewicht des Copolymeren, nicht übersteigt.

5. Zusammensetzung nach einem der vorhergehenden Ansprüche, dadurch gekennzeichnet, daß das fixierende und/oder konditionierende Polymer in einer Konzentration von 0,1 bis 20 Gew.-%, bezogen auf das Gesamtgewicht der Zusammensetzung, vorliegt.

6. Zusammensetzung nach Anspruch 5, dadurch gekennzeichnet, daß das fixierende und/oder konditionierende Polymer in einer Konzentration von 1 bis 10 Gew.-%, bezogen auf das Gesamtgewicht der Zusammensetzung, vorliegt.

7. Zusammensetzung nach einem der vorhergehenden Ansprüche, dadurch gekennzeichnet, daß die Konzentration des Acrylcopolymeren im Bereich von 0,01 bis 15 Gew.-%, bezogen auf das Gesamtgewicht der Zusammensetzung, liegt.

8. Zusammensetzung nach Anspruch 7, dadurch gekennzeichnet, daß die Konzentration des Acrylcopolymeren im Bereich von 0,05 bis 8 Gew.-%, bezogen auf das Gesamtgewicht der Zusammensetzung, liegt.

9. Zusammensetzung nach einem der vorhergehenden Ansprüche, dadurch gekennzeichnet, daß das fixierende und/oder konditionierende Polymer unter anionischen, kationischen, amphoteren und nichtionischen Polymeren sowie deren Gemischen ausgewählt ist.

10. Zusammensetzung nach Anspruch 9, dadurch gekennzeichnet, daß das anionische Polymer unter folgenden Polymeren ausgewählt ist:
- Copolymeren von Acrylsäure, wie Acrylsäure-Ethylacrylat-N-tert-Butylacrylamid-Terpolymeren;
- von Crotonsäure abgeleiteten Copolymeren, wie Vinylacetat-Vinyl-tert-butylbenzoat-Crotonsäure-Terpolymeren und Crotonsäure-Vinylacetat-Vinylneododecanat-Terpolymeren;
- von Maleinsäure, Fumarsäure, Itaconsäure beziehungsweise den Anhydriden dieser Säuren abgeleiteten Copolymeren mit Vinylestern, Vinylethern, Vinylhalogeniden, Phenylvinylderivaten und Acrylsäure und ihren Estern, wie Methylvinylether-Maleinsäureanhydrid-monoester-Copolymeren;
- Copolymeren von Methacrylsäure mit Methylmethacrylat;
- Copolymeren von Methacrylsäure mit Ethylacrylat;
- Vinylpyrrolidon-Acrylsäure-Laurylmethacrylat-Terpolymeren
und
- Methacrylsäure-Ethylacrylat-tert-Butylacrylat-Terpolymeren.

11. Zusammensetzung nach Anspruch 9, dadurch gekennzeichnet, daß das kationische Polymer unter folgenden Polymeren ausgewählt ist:
- mit Dimethylsulfat quaternisierten Copolymeren von Acrylamid mit Dimethylaminoethylmethacrylat;
- Copolymeren von Acrylamid mit [2-(Methacryloyloxy)-ethyl]-trimethylammoniumchlorid;
- Copolymeren von Acrylamid mit [2-(Methacryloyloxy)-ethyl]-trimethylammoniummethosulfat;
- gegebenenfalls quaternisierten Copolymeren von Vinylpyrrolidon mit Dialkylaminoalkylacrylaten und -methacrylaten;
- Dimethylaminoethyl-methacrylat-Vinylcaprolactam-Vinylpyrrolidon-Terpolymeren
und
- quaternisierten Vinylpyrrolidon-Dimethylaminopropyl-methacrylamid-Copolymeren.

12. Zusammensetzung nach Anspruch 9, dadurch gekennzeichnet, daß das amphotere Polymer ausgewählt ist unter Polymeren mit Einheiten, die sich ableiten von:
a) mindestens einem Monomeren, das unter Acrylamiden und Methacrylamiden, die am Stickstoff mit einer Alkylgruppe substituiert sind, ausgewählt ist,
b) mindestens einem sauren Comonomeren, das eine oder mehrere reaktive Carboxylgruppen enthält,
und
c) mindestens einem basischen Comonomeren, wie Acrylsäure- und Methacrylsäureestern mit primären, sekundären, tertiären oder quartären Aminsubstituenten und dem Produkt der Quaternisierung von Dimethylaminoethyl-methacrylat mit Dimethylsulfat oder Diethylsulfat.

13. Zusammensetzung nach Anspruch 12, dadurch gekennzeichnet, daß das amphotere Polymer unter den nach CTFA als Octylacrylamid-Acrylate-Butylaminoethylmethacrylat-Copolymere bezeichneten Copolymeren ausgewählt ist.

14. Zusammensetzung nach einem der vorhergehenden Ansprüche, dadurch gekennzeichnet, daß sie in Form eines Aerosols unter Druck steht.

15. Zusammensetzung nach Anspruch 14, dadurch gekennzeichnet, daß sie, bezogen auf das Gesamtgewicht der Zusammensetzung, 10 bis 50 Gew.-% eines Treibmittels enthält.

16. Verwendung eines Acrylcopolymeren nach einem der Ansprüche 1 bis 4 zur Verbesserung der Eigenschaften von kosmetischen Zusammensetzungen, die ein fixierendes und/oder konditionierendes Polymer enthalten, beim Zerstäuben und/oder Versprühen.

17. Verwendung eines Acrylcopolymeren wie in einem der Ansprüche 1 bis 4 beschrieben zur Verbesserung der Eigenschaften von als Aerosol unter Druck stehenden kosmetischen Zusammensetzungen, die ein fixierendes und/oder konditionierendes Polymer enthalten, beim Zerstäuben und/oder Versprühen.

18. Verfahren zur kosmetischen Behandlung keratinhaltiger Materialien, wie der Haare, dadurch gekennzeichnet, daß es darin besteht, eine kosmetische Zusammensetzung nach einem der Ansprüche 1 bis 15 auf die keratinhaltigen Materialien aufzutragen und gegebenenfalls, unter Umständen nach einer Einwirkzeit, mit Wasser auszuspülen.
